(19) Europäisches Patentamt
European Patent Office
Office européen des brevets

(11) EP 0 587 837 B1

(12) # FASCICULE DE BREVET EUROPEEN

(45) Date de publication et mention de la délivrance du brevet:
**03.06.1998 Bulletin 1998/23**

(21) Numéro de dépôt: **93905443.3**

(22) Date de dépôt: **02.03.1993**

(51) Int. Cl.$^6$: **A61K 7/42**, A61K 7/48, A61K 7/13

(86) Numéro de dépôt international:
**PCT/FR93/00207**

(87) Numéro de publication internationale:
**WO 93/17659 (16.09.1993 Gazette 1993/22)**

(54) **COMPOSITION COSMETIQUE CONTENANT DES PIGMENTS MELANIQUES EN ASSOCIATION AVEC CERTAINS TOCOPHEROLS, ET PROCEDE DE PROTECTION DE LA PEAU, DES CHEVEUX, DES MUQUEUSES ET DES COMPOSITIONS COSMETIQUES**

Melanin-Pigmente zusammen mit Tocopherol enthaltende kosmetische Zusammensetzung und Verfahren zum Schutz der Haut, des Haares

Cosmetic composition containing melanistic pigments in association with certain tocopherols, and method for protecting the skin, the hair, the mucous membranes and cosmetic compositions

(84) Etats contractants désignés:
**DE ES FR GB IT**

(30) Priorité: **03.03.1992 FR 9202532**

(43) Date de publication de la demande:
**23.03.1994 Bulletin 1994/12**

(73) Titulaire: **L'OREAL**
**75008 Paris (FR)**

(72) Inventeurs:
- **HANSENNE, Isabelle**
  **F-75017 Paris (FR)**
- **FORESTIER, Serge**
  **F-77410 Claye-Souilly (FR)**
- **N'GUYEN, Quang, Lan**
  **F-92160 Antony (FR)**

(74) Mandataire: **Casalonga, Axel et al**
**BUREAU D.A. CASALONGA - JOSSE**
**Morassistrasse 8**
**80469 München (DE)**

(56) Documents cités:

| | |
|---|---|
| **EP-A- 0 376 776** | **EP-A- 0 456 545** |
| **EP-A- 0 467 767** | **FR-A- 2 666 226** |
| **GB-A- 2 207 153** | **US-A- 4 144 325** |

Remarques:
Le dossier contient des informations techniques présentées postérieurement au dépôt de la demande et ne figurant pas dans le présent fascicule.

EP 0 587 837 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Printed by Xerox (UK) Business Services
2.16.3/3.4

## Description

La présente invention a pour objet une composition cosmétique comme revendiquée renfermant des pigments mélaniques en association avec certains tocophérols, visant notamment la protection cosmétique de l'épiderme humain, des muqueuses et des cheveux contre les radicaux libres ainsi qu'un procédé de protection cosmétique de l'épiderme humain, des muqueuses et des cheveux et des compositions cosmétiques contre ces radicaux.

Le rayonnement solaire, la chaleur, la pollution atmosphérique et notamment les fumées et le tabac sont connus pour entrainer la formation de radicaux libres. Ces radicaux libres amorcent des réactions de dégradation des lipides, protéines, acides nucléiques qui sont présents notamment dans la peau, les muqueuses et les cheveux. De plus, les compositions cosmétiques comportent des constituants sensibles aux radicaux libres et notamment des lipides.

Il est donc apparu particulièrement important de protéger la peau, les muqueuses, et les cheveux de ces radicaux libres.

La vitamine E est un antioxydant connu pour protéger les phospholipides de la membrane cellulaire (J.B. CHAZAN et M. SZULC - Radicaux Libres et Vitamine E - Cah. Nutr. Diet. 1987 - XXII - 1 - pages 66-76) et ses propriétés en tant qu'agents antiradicaux libres (ARL) sont connues.

Par ailleurs, certains pigments mélaniques ont déjà été utilisés dans des compositions cosmétiques pour la protection de l'épiderme humain contre les UV, le maquillage de la peau, des cils et des sourcils ou encore la coloration des cheveux où leur rôle protecteur vis-à-vis du rayonnement solaire a été établi.

Selon l'invention, on a découvert de façon surprenante que l'association de pigments mélaniques avec certains tocophérols, dans une composition cosmétique, permet d'obtenir de très bons résultats en termes d'activité antiradicaux libres. L'activité antiradicaux libres de l'association est en effet plus active que chacun des composants, la vitamine E d'une part ou les pigments mélaniques d'autre part, quand ceux-ci sont pris séparément, pour des concentrations comparables.

La présente invention a donc pour objet une composition cosmétique comprenant l'association de certains tocophérols avec au moins un pigment mélanique, dans un support cosmétiquement acceptable.

Les tocophérols selon l'invention sont choisis parmi l'$\alpha$-tocophérol, le $\beta$-tocophérol, le $\gamma$-tocophérol et le $\delta$-tocophérol ainsi que leurs isomères. L'$\alpha$-tocophérol, préféré selon l'invention, est la vitamine E. Il existe sous les formes D-$\alpha$-tocophérol, L-$\alpha$-tocophérol et DL-$\alpha$-tocophérol. Du DL-$\alpha$-tocophérol est commercialisé notamment par la Société HOFFMANN LA ROCHE. Un mélange d'$\alpha$-tocophérol, de $\beta$-tocophérol, de $\gamma$-tocophérol et de $\delta$-tocophérol dans un rapport 25/25/25/25 dissous dans de l'huile de soja à 50% est commercialisé sous la dénomination "TOCOPHEROL Concentrat Naturel" par la Société ROSSOW.

L'activité antiradicaux libres, améliorée pour l'association selon l'invention par rapport à l'activité de la vitamine E d'une part ou celle des pigments mélaniques d'autre part, a été mise en évidence par la méthode "Head Space" (NGUYEN QL et coll. Symposium of AFECG-SFC, Bordeaux mai 1984, p. 358-359, Evaluation de l'oxydation aldéhydique dans les produits cosmétiques; WARNER K et Coll. Journal Of Food Science- 1974 V39- p 761-765, Pentane formation and rancidity in vegetable oils). Cette méthode permet d'évaluer le pourcentage d'inhibition de l'oxydation de la vitamine F, elle-même très oxydable, par le produit à tester.

Les pigments mélaniques ont un diamètre moyen compris entre 10 nm et 50 000 nm, de préférence entre 30 nm et 20 000 nm.

Le (ou les) pigment(s) mélanique(s) sont dérivés de sources naturelles ou synthétiques et peuvent être obtenus (A) par oxydation d'au moins un composé indolique, (B) par polymérisation oxydante ou enzymatique de précurseurs mélaniques, (C) par extraction de la mélanine à partir de substances naturelles en contenant ou (D) par culture de microorganismes produisant de la mélanine.

Les tocophérols de l'invention sont présents dans la composition cosmétique selon l'invention, seuls ou en mélange, à une concentration comprise entre 0,02 et 10% en poids par rapport au poids total de la composition, et de préférence comprise entre 0,02% et 6%.

Les pigments mélaniques sont avantageusement présents dans la composition cosmétique selon l'invention à une concentration comprise entre 0,001 et 2%, de préférence entre 0,005 et 0,5% en poids par rapport au poids total de la composition.

Le rapport pondéral tocophérol/pigment mélanique peut être prévu entre 0,1 et 100, de préférence 0,5 à 50.

Selon l'invention, les pigments peuvent être choisis parmi les pigments mélaniques obtenus par oxydation d'un composé indolique, par polymérisation oxydante ou enzymatique de précurseurs mélaniques, par extraction de la mélanine de substances naturelles ou par culture de microorganismes produisant de la mélange.

(A) Les pigments mélaniques peuvent, en premier lieu, être obtenus par oxydation d'au moins un composé indolique choisi notamment parmi ceux répondant à la formule (I) :

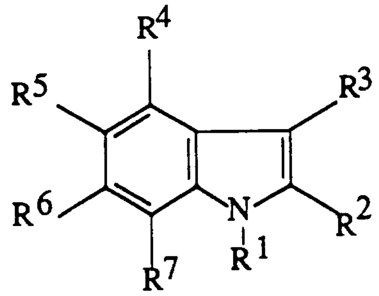

(I)

dans laquelle :

- $R^1$ et $R^3$ représentent, indépendamment l'un de l'autre, un atome d'hydrogène ou un groupe alkyle en $C_1$-$C_4$;
- $R^2$ représente un atome d'hydrogène, un groupe alkyle en $C_1$-$C_4$, un groupe carboxyle ou un groupe alcoxy ($C_1$-$C_4$)-carbonyle;
- $R^4$ et $R^7$ représentent, indépendamment l'un de l'autre, un atome d'hydrogène, un groupe hydroxy, un groupe alkyle en $C_1$-$C_4$, un groupe amino, un groupe alcoxy en $C_1$-$C_4$, un groupe acyl ($C_2$-$C_4$)-oxy ou un groupe acyl ($C_2$-$C_4$)-amino;
- $R^5$ représente un atome d'hydrogène, un groupe hydroxy, un groupe alcoxy en $C_1$-$C_4$, un groupe alkyle en $C_1$-$C_4$, un atome d'halogène, un groupe amino, un groupe acyl ($C_2$-$C_{14}$)- oxy, un groupe acyl ($C_2$-$C_4$)-amino ou un groupe triméthylsilyloxy;
- $R^6$ représente un atome d'hydrogène, un groupe hydroxy, un groupe alcoxy en $C_1$-$C_4$, un groupe amino, un groupe acyl ($C_2$-$C_4$)-oxy, un groupe acyl ($C_2$-$C_4$)-amino, un groupe triméthylsilyloxy ou un groupe hydroxyalkyl ($C_2$-$C_4$)-amino;
- $R^5$ et $R^6$ pouvant également former, conjointement avec les atomes de carbone auxquels ils sont rattachés, un cycle méthylènedioxy éventuellement substitué par un groupement alkyle en $C_1$-$C_4$ ou alcoxy en $C_1$-$C_4$, ou bien un cycle carbonyldioxy;
- au moins l'un des radicaux $R^4$ à $R^7$ représente un groupement OZ ou $NHR^0$, l'un au plus des radicaux $R^4$ à $R^7$ représentant $NHR^0$ et deux au plus des radicaux $R^4$ à $R^7$ représentant OZ et, dans le cas où Z représente un atome d'hydrogène, les deux groupes OH sont dans les positions 5 et 6; et au moins l'un des radicaux $R^4$ à $R^7$ représente un atome d'hydrogène, et dans le cas où un seul de ces radicaux représente un atome d'hydrogène, un seul radical parmi les radicaux $R^4$ à $R^7$ représente alors $NHR^0$ ou OZ, les autres radicaux représentant un groupe alkyle en $C_1$-$C_4$;
- le radical $R^0$ du groupement $NHR^0$ désignant un atome d'hydrogène, un groupe acyle en $C_2$-$C_4$ ou hydroxyalkyle en $C_2$-$C_4$, et le radical Z du groupement OZ désignant un atome d'hydrogène, un groupe acyle en $C_2$-$C_{14}$, un groupe alkyle en $C_1$-$C_4$, ou un groupe triméthylsilyle,

et leurs sels de métaux alcalins, alcalino-terreux, d'ammonium ou d'amines.

Les composés indoliques de formule (I) ci-dessus sont choisis, de préférence, parmi le 4-hydroxyindole, le 5-hydroxyindole, le 6-hydroxyindole, le 7-hydroxyindole, le 4-hydroxy-5-méthoxyindole, le 4-hydroxy 5-éthoxyindole, le 2-carboxy 5-hydroxyindole, le 5-hydroxy 6-méthoxyindole, le 6-hydroxy 7-méthoxyindole, le 5-méthoxy 6-hydroxyindole, le 5,6-dihydroxyindole, le N-méthyl 5,6-dihydroxyindole, le 2-méthyl 5,6-dihydroxyindole, le 3-méthyl 5,6-dihydroxyindole, le 2,3-diméthyl 5,6-dihydroxyindole, le 2-carboxy 5,6-dihydroxyindole, le 4-hydroxy 5-méthyl indole, le 2-carboxy 6-hydroxyindole, le 6-hydroxy N-méthylindole, le 2-éthoxycarbonyl 5,6-dihydroxythdole, le 4-hydroxy 7-méthoxy 2,3-diméthylindole, le 4-hydroxy 5-éthoxy N-méthylindole, le 6-hydroxy 5-méthoxy 2-méthylindole, le 6-hydroxy 5-méthoxy 2,3-diméthylindole, le 6-hydroxy 2-éthoxycarbonylindole, le 7-hydroxy 3-méthylindole, le 5-hydroxy 6-méthoxy 2,3-diméthylindole, le 5-hydroxy 3-méthylindole, le 5-acétoxy 6-hydroxyindole, le 5-hydroxy 2-éthoxycarbonylindole, le 6-hydroxy 2-carboxy 5-méthylindole, le 6-hydroxy 2-éthoxycarbonyl 5-méthoxyindole, le 6-N-β-hydroxyéthylaminoindole, le 4-aminoindole, le 5-aminoindole, le 6-aminoindole, le 7-aminoindole, le N-méthyl 6-β-hydroxyéthylaminoindole, le 6-amino 2,3-diméthylindole, le 6-amino 2,3,4,5-tétraméthylindole, le 6-amino 2,3,4-triméthylindole, le 6-amino 2,3,5-triméthylindole, le 6-amino 2,3,6-triméthylindole, le 5,6,-diacétoxyindole, le 5-méthoxy 6-acétoxyindole, le 5,6-diméthoxyindole, le 5,6-méthylènedioxyindole, le 5,6-triméthylsilyloxyindole, l'ester phosphorique du 5,6-dihydroxyindole, le 5,6-dibenzyloxyindole, et les sels d'addition de ces composés.

Le 5,6-dihydroxyindole est particulièrement préféré.

L'oxydation du composé indolique de formule (I) peut s'effectuer en milieu aqueux ou eau-solvant(s), à l'air, en présence ou non d'un agent alcalin et/ou d'un catalyseur métallique d'oxydation tel que par exemple, l'ion cuivrique.

Le milieu réactionnel est, de préférence, constitué par de l'eau et peut, le cas échéant, être constitué par un mélange d'eau et d'au moins un solvant choisi de telle façon qu'il solubilise rapidement le composé indolique de formule (I). Parmi ces solvants, on peut citer, à titre d'exemples, les alcools inférieurs en $C_1$-$C_4$, tels que l'alcool éthylique, l'alcool propylique ou isopropylique, l'alcool tert.-butylique, les alkylèneglycols, tels que l'éthylèneglycol, le propylèneglycol, les alkyléthers d'alkylèneglycols, tels que les éthers monométhylique, monoéthylique et monobutylique de l'éthylèneglycol, les monométhyléthers du propylèneglycol et du dipropylèneglycol, et le lactate de méthyle.

L'oxydation peut également s'effectuer en mettant en oeuvre le peroxyde d'hydrogène en présence d'un agent alcalin, tel que, de préférence, l'ammoniaque, ou en présence d'un ion iodure, l'iodure étant, de préférence, l'iodure d'un métal alcalin, alcalino-terreux ou d'ammonium.

On peut également procéder' à l'oxydation en utilisant l'acide périodique et ses sels hydrosolubles et dérivés, les permanganates et bichromates, tels que de sodium ou de potassium, l'hypochlorite de sodium, le ferricyanure de potassium, le persulfate d'ammonium, l'oxyde d'argent, l'oxyde de plomb, le chlorure ferrique, le nitrite de sodium, les sels de terres rares dont, notamment le cérium, et des oxydants organiques choisis parmi les ortho- et parabenzoquinones, les ortho- et parabenzoquinones mono- ou diimines, les 1,2- et 1,4-naptoquinones, les 1,2- et 1,4-naphtoquinones mono- ou diimines telles que définies dans la demande EP-A-0 376 776. Le sel d'acide periodique préféré est le periodate de sodium.

Il est possible d'activer les agents oxydants par un modificateur de pH.

On peut également envisager une oxydation enzymatique.

Le produit insoluble est isolé par filtration, centrifugation, lyophilisation ou atomisation; il est ensuite broyé ou micronisé pour atteindre la granulométrie désirée.

(B) Les pigments mélaniques selon l'invention peuvent également provenir de la polymérisation oxydante ou enzymatique de précurseurs mélaniques, tels que la L-tyrosine, la L-dopa, le catéchol et leurs dérivés.

(C) Les pigments mélaniques selon l'invention peuvent aussi provenir de l'extraction de la mélanine de substances naturelles telles que les cheveux humains, l'encre de céphalopodes (seiches, poulpes), encore connue sous le nom de sépiomélanine, auquel cas le pigment est broyé et purifié avant son utilisation.

(D) Les pigments mélaniques selon l'invention peuvent enfin être obtenus par culture de microorganismes. Ces microorganismes produisent de la mélanine soit naturellement, soit par modification génétique ou par mutagénèse. Des modes de préparations de ces mélanines sont décrits par exemple dans la demande de brevet WO90/04029.

Le (ou les) pigment(s) mélanique(s) peut ou (peuvent) être présent(s) à la surface ou incorporé(s) dans une charge particulaire inerte, minérale ou organique, pour constituer un pigment mélanique composite synthétique formé in situ. Dans ce cas, le (ou les) pigment(s) mélanique(s) peut (ou peuvent) résulter de l'oxydation d'au moins un composé indolique de formule (I), telle que définie ci-dessus, en mélange avec la charge, dans un milieu essentiellement non-solvant de ladite charge, à une température pouvant aller de la température ambiante jusqu'à environ 100°C, ou encore résulter de la polymérisation oxydante de précurseurs mélaniques sur la charge.

Les conditions générales de l'oxydation des composés indoliques de formule (I) sont les mêmes que celles mentionnées ci-dessus.

Selon un premier mode de réalisation, la charge particulaire est une charge minérale inerte constituée avantageusement de particules de granulométrie inférieure à 20 000 nm. De tels pigments mélaniques composites, déposés sur charge minérale, sont décrits, ainsi que leur préparation, dans la demande de brevet français FR-A-2 618 069. Selon un deuxième mode de réalisation de la présente invention, la charge particulaire est une charge polymérique inerte, choisie avantageusement parmi les polymères naturels ou synthétiques, organiques ou inorganiques, à réseau réticulé, cristallin ou amorphe, ayant un poids moléculaire compris entre 5000 et 5 000 000. Des pigments mélaniques composites sur charge polymérique ainsi que leur préparation sont décrits dans le brevet luxembourgeois n° 87 429.

Les polymères organiques ou synthétiques sont, en particulier, choisis parmi les polymères dérivés de la kératine, de la fibroïne de soie, de la chitine ou de la cellulose, ou parmi les polyamides ou les homo- ou copolymères résultant de la polymérisation de monomères mono- ou polyéthyléniques, aliphatiques ou aromatiques, à réseau réticulé, cristallin ou amorphe.

Les polymères cellulosiques sont choisis plus particulièrement parmi les celluloses microcristallines, telles que les produits vendus sous la dénomination "AVICEL" par la société FMC CORPORATION.

Parmi les polymères synthétiques, on peut tout particulièrement citer le polyéthylène, le polypropylène, le polystyrène, le poly(méthacrylate de méthyle) vendus sous les dénominations "MICROPEARL M" et "MICROPEARL M100" par la société SEPPIC, le poly(méthacrylate de méthyle) réticulé, tel que le produit vendu sous la dénomination de "MICROPEARL M 305" par la société SEPPIC. D'autres polymères sont, en particulier, choisis parmi la poly-β-alanine réticulée, telle que décrite dans le brevet français 2 530 250 dont le taux de réticulation est compris entre 1 et 15% et,

de préférence, entre 1 et 8%.

On peut également utiliser, à titre de polymères, des produits connus sous la dénomination de microéponges, tels que des polymères réticulés de styrène/divinylbenzène ou de méthacrylate de méthyle/diméthacrylate d'éthylène-glycol ou de stéarate de vinyle/divinylbenzène, tels que décrits dans les brevets WO-88/01164 et US-A-4 690 825. De tels polymères sont essentiellement constitués par des billes de polymères réticulés comprenant un réseau interne de pores, capable de retenir le pigment mélanique.

Selon un troisième mode de réalisation, la charge particulaire est une charge constituée par des particules à structure lamellaire, organiques ou minérales, qui ont une dimension inférieure à 50 000 nm. Les particules de structure lamellaire sont constituées de feuillets pour lesquels le rapport entre la plus grande dimension et l'épaisseur est notamment compris entre 2 et 100.

Des pigments mélaniques composites, déposés sur une charge lamellaire ainsi que leur préparation sont décrits dans la demande de brevet européen n° 0467767.

La composition cosmétique de l'invention peut être utilisée comme composition protectrice de l'épiderme humain, des muqueuses ou des cheveux, comme produit de maquillage de la peau ou de ses phanères. L'association tocophérol/pigment mélanique est également utile pour la protection des compositions cosmétiques elles-mêmes contre les radicaux libres; elle est aussi utile dans les compositions à usage bucco-dentaire, telles que les pâtes dentifrices.

Cette composition peut se présenter en particulier sous forme de lotion, de lotion épaissie, de gel, de dispersion vésiculaire, de dispersion de nanoparticules, de crème, de lait, de poudre, de pommade, de bâtonnet solide et éventuellement être conditionnée en aérosol et se présenter sous forme de mousse ou de spray.

Les composition conformes à l'invention peuvent se présenter sous forme de dispersion vésiculaire de lipides amphiphiles ioniques ou non-ioniques. Elles sont préparées notamment en faisant gonfler les lipides dans une solution aqueuse pour former des sphérules dispersées dans le milieu aqueux comme décrit dans l'article BANGHAM, STANDISH & WATKINS, J. Mol. Biol., 13,238 (1965) ou dans les brevets FR-2 315 991 et 2 416 008 de la demanderesse.

Les différents types de procédés de préparation sont décrits dans "Les liposomes en biologie cellulaire et pharmacologie", Edition INSERM/John Liberry Eutotext, 1987, pages 6 à 18.

Les compositions peuvent se présenter sous forme de dispersions de nanoparticules. Le terme "nanoparticules" recouvre d'une part les nanosphères et, d'autre part, les nanocapsules; on désigne par le terme "nanosphères" les nanoparticules constituées par une matrice polymérique poreuse sur laquelle le principe actif est absorbé et/ou adsorbé et par le terme "nanocapsules" les nanoparticules constituées par une membrane polymérique, qui entoure un coeur formé par le principe actif. De telles formes de composition sont décrites par exemple dans les demandes de brevet EP-274 961 et FR-2 659 554.

Elle peut contenir les adjuvants cosmétiques habituellement utilisés tels que des corps gras, des solvants organiques, des silicones, des épaississants, des adoucissants, des filtres solaires UV-A, UV-B, ou à bande large, des agents anti-mousses, des agents hydratants, des parfums, des conservateurs, des tensio-actifs, des charges, des séquestrants, des polymères anioniques, cationiques, non-ioniques, amphotères, ou leurs mélanges, des propulseurs, des agents alcalinisants ou acidifiants, des colorants, des pigments d'oxydes métalliques comme les oxydes de fer, ou tout autre ingrédient habituellement utilisé en cosmétique, ainsi que des agents autobronzants comme la dihydroxyacétone.

Parmi les solvants oganiques, on peut citer les alcools et polyols inférieurs tels que l'éthanol, l'isopropanol, le propylène glycol, la glycérine et le sorbitol. Les esters d'acides gras tels que le myristate d'isopropyle, l'adipate d'isopropyle, les benzoates d'alcools gras en $C_{12}C_{15}$ ("FINSOLV TN" de FINETEX), l'alcool myristique oxypropyléné à 3 moles d'oxyde de propylène ("WITCONOL APM" de WITCO), etc. les triglycérides d'acides caprique et caprylique ("MIGLYOL 812" de HULS)

La composition cosmétique selon l'invention peut donc contenir des épaississants qui peuvent être choisis parmi les polymères d'acide acrylique réticulés ou non, et particulièrement les acides polyacryliques réticulés par un agent polyfonctionnel tels que les produits vendus sous la dénomination "CARBOPOL" par la société GOODRICH, les dérivés de cellulose tels que la méthylcellulose, l'hydroxyéthylcellulose, l'hydroxypropylméthylcellulose, les sels de sodium de la carboxyméthylcellulose, les mélanges d'alcool cétylstéarylique et d'alcool cétylstéarylique oxyéthyléné à 33 moles d'oxyde d'éthylène.

On peut également utiliser les produits résultant de l'interaction ionique d'un polymère cationique constitué par un copolymère de cellulose ou d'un dérivé de cellulose greffés par un sel de monomère hydrosoluble d'ammonium quaternaire et d'un polymère anionique carboxylique tels que décrits dans le brevet français FR-2 598 611. On utilise de préférence le produit d'interaction ionique d'un copolymère d'hydroxyéthylcellulose greffé par voie radicalaire par du chlorure de diallydiméthylammonium tel que le polymère commercialisé sous la dénomination "CELQUAT L 200" par la société National Starch avec, soit des copolymères d'éthylène et d'anhydride maléique tels que les produits vendus sous la dénomination "EMA 31" par la société MONSANTO, soit des copolymères 50/50 d'acide méthacrylique et de méthacrylate de méthyle.

Un autre produit de ce type utilisable est le produit résultant de l'interaction ionique du copolymère d'hydroxyéthyl cellulose greffé par voie radicalaire par du chlorure de diallyldiméthylammonium avec un polymère anionique carboxylique réticulé tel que les copolymères de l'acide méthacrylique et de l'acrylate d'éthyle réticulés vendus sous la dénomination "VISCOATEX" 538 ou 46 par la société COATEX.

Les corps gras peuvent être constitués par une huile ou une cire ou leur mélange, les acides gras, les alcools gras, la vaseline, la paraffine, la lanoline, la lanoline hydrogénée, la lanoline acétylée, le beurre de cacao ou de karité.

Les huiles sont choisies parmi les huiles animales, végétales, minérales ou de synthèse et notamment l'huile de palme hydrogénée, l'huile de ricin hydrogénée, l'huile de vaseline, l'huile de paraffine, l'huile de Purcellin, les huiles de silicone et les isoparaffines.

Les cires sont choisies parmi les cires animales, fossiles, végétales, minérales ou de synthèse. On peut citer notamment les cires d'abeille, les cires de Carnauba, de Candelila, de canne à sucre, du Japon, les ozokérites, la cire de Montan, les cires microcristallines, les paraffines, les cires et résines de silicone.

Lorsque la composition cosmétique selon l'invention est utilisée pour la protection de l'épiderme humain, elle peut se présenter sous forme de suspension ou de dispersion dans des solvants ou des corps gras, ou encore sous forme d'émulsion telle qu'une crème ou un lait, sous forme de pommade, de gel, de bâtonnet solide ou de mousse aérosol.

Les émulsions peuvent contenir en outre des agents tensio-actifs anioniques, non-ioniques, cationiques ou amphotères.

La composition pour la protection de l'épiderme humain contre les radicaux libres peut en outre contenir des agents antisolaires ou autobronzants et, dans les mêmes formes que ci-dessus, être utilisée comme composition antisolaire ou autobronzante protectrice.

Lorsque la composition cosmétique selon l'invention est utilisée pour la protection des cheveux, elle peut se présenter sous forme de shampooing, de lotion, de gel ou composition à rincer, à appliquer avant ou après shampooing, avant ou après coloration ou décoloration, avant, pendant ou après permanente ou défrisage, de lotion ou gel coiffants ou traitants, de lotion ou gel pour le brushing ou la mise en plis, de laque pour cheveux, de composition de permanente ou de défrisage, de coloration ou décoloration des cheveux.

Lorsque la composition est utilisée comme produit de maquillage des cils, des sourcils ou de la peau, tel que crème de traitement de l'épiderme, fond de teint, bâton de rouge à lèvres, fard à paupières, fard à joues, ligneur encore appelé "eye-liner", ou mascara, eue peut se présenter sous forme solide ou pâteuse, anhydre ou aqueuse, comme des émulsions huile-dans-eau ou eau-dans-huile ou encore des suspensions.

Lorsque la composition est utilisée pour le maquillage des cheveux, elle peut également se présenter sous forme solide ou pâteuse, anhydre ou aqueuse, sous forme d'émulsions huile-dans-eau ou eau-dans-huile ou encore sous forme de suspensions ou de gels, comme des gels de coiffage ou de maquillage.

Lorsque la composition est utilisée dans une application buccodentaire, elle peut comprendre des adjuvants et additifs usuels pour les compositions à usage buccal et notamment des agents tensio-actifs, des agents épaississants, des agents humectants et des agents de polissage tels que la silice, des agents actifs comme les fluorures, et en particulier le fluorure de sodium et éventuellement des agents édulcorants comme le saccharinate de sodium.

L'invention a aussi pour objet un procédé de protection de l'épiderme humain, des muqueuses, et des cheveux contre l'action néfaste des radicaux libres consistant à appliquer sur ceux-ci une quantité efficace de la composition ci-dessus.

L'invention a également pour objet un procédé de maquillage consistant à appliquer la composition cosmétique ci-dessus sur la peau, ses phanères ou les cheveux.

L'invention a également pour objet un procédé de protection des compositions cosmétiques contre les radicaux libres consistant à y incorporer une quantité efficace de l'association tocophérol/pigment mélanique.

L'invention sera mieux illustrée par les exemples non limitatifs ci-après.

**EXEMPLE 1**

On prépare une crème solaire de composition suivante :

| | |
|---|---|
| - Mélanine obtenue par oxydation du 5,6-dihydroxyindole | 0,025 g MA |
| - DL-α-tocophérol (Vitamine E) vendu par la Société HOFFMANN LA ROCHE | 0,5 g |
| - p-méthoxycinnamate de 2-éthylhexyle vendu sous la dénomination de "PARSOL MCX" par la société GIVAUDAN | 5,0 g |
| - Mélange((80/20) d'alcool cérylstéarylique et d'alcool cétylstéarylique oxyéthyléné à 33 moles d'oxyde d'éthylène vendu sous la dénomination "SINNOWAX AO" par la société HENKEL | 7,0 g |

(suite)

| | |
|---|---|
| - Mono et distéarate de glycérol (40/50) vendu sous la dénommination "GELEOL par la société GATTEFOSSE | 2,0 g |
| - Alcool cétylique (90% $C_{16}$) | 1,5 g |
| - Huile de vaseline | 15,0 g |
| - Polydiméthylsiloxane | 1,5 g |
| - Glycérine | 3,0 g |
| - Conservateurs, parfum qs | |
| - Eau qsp | 100 g |

**EXEMPLE 2**

On prépare une crème autobronzante de composition suivante :

| | |
|---|---|
| - Mélanine obtenue par oxydation du 5,6-dihydroxyindole | 0,015 g MA |
| - DL-$\alpha$-tocophérol (Vitamine E) vendu par la Société HOFFMANN LA ROCHE | 1,0 g |
| - Dihydroxyacétone | 3,0 g |
| - Mélange(80/20) d'alcool cérylstéarylique et d'alcool cétylstéarylique oxyéthyléné à 33 moles d'oxyde d'éthylène vendu sous la dénomination "SINNOWAX AO" par la société HENKEL | 7,0 g |
| - Mono et distéarate de glycérol (40/50) vendu sous la dénomination"GELEOL" par la société GATTEFOSSE | 2,0 g |
| - Alcool cétylique (90% $C_{16}$) | 1,5 g |
| - Huile de vaseline | 10,0 g |
| - Polydiméthylsiloxane | 1,5 g |
| - Glycérine | 10,0 g |
| - Conservateurs, parfum qs | |
| - Eau qsp | 100 g |

**EXEMPLE 3**

On prépare un gel de maquillage pour cheveux de composition suivante :

| | |
|---|---|
| - Copolymère acide méthacrylique/acrylate d'éthyle réticulé en dispersion anionique à 38% MA dans l'eau, commercialisé sous la dénomination "VISCOATEX 538" par la Société COATEX | 1,6 g MA |
| - Copolymère hydroxyéthylcellulose/chlorure de diallyldiméthyl ammonium, commercialisé sous la dénomination "CELQUAT L 200" par la Société NATIONAL STARCH | 1,6 g |
| - Polydiméthylsiloxane à groupements amminoéthylpropylamine en émulsion cationique à 35% MA dans l'eau, commercialisé sous la dénomination "DC 929" par la Société DOW CORNING | 0,3 g MA |
| - Ethanol | 10,0 g |
| - DL-$\alpha$ tocophérol (Vitamin E) vendu par la Société HOFFMANN LA ROCHE | 0,1 g |
| - Pigment mélanique obtenu par oxydation du 5,6-dihydroxyindole | 1,0 g |
| - Conservateur, séquestrant qs | |

(suite)

| | |
|---|---|
| - Parfum qs | |
| - 2-amino, 2-méthyl, 1-propanol qsp pH 7,5 | |
| - Eau déminéralisée qsp | 100 g |

**EXEMPLE 4**

On prépare une pâte dentifrice ayant la composition suivante :

| | |
|---|---|
| - Silice commercialisée sous la dénomination "TIXOSIL 73" par la Société RHONE POULENC | 12,0 g |
| - Silice commercialisée sous la dénomination "TIXOSIL 333" par la Société RHONE POULENC | 8,0 g |
| - Sorbitol en solution à 70% MA dans l'eau | 14,0 g MA |
| - Glycérine | 5,0 g |
| - Carboxyméthylcellulose vendue sous la dénomination "BLANOSE 9M 31 F" par la Société HERCULES | 1,4 g |
| - Lauryl sulfate de sodium à 92% de MA dans l'eau vendu sous la dénomination "EMPICOL LZV/E" par la Société MARCHON | 1,8 g |
| - Fluorure de sodium | 0,22 g |
| - Dioxyde de titane | 0,5 g |
| - Saccharinate de sodium qs | |
| - Arôme qs | |
| - Vitamine E | 0,15 g |
| - Pigment mélanique obtenu par oxydation du 5,6-dihydroxyindole | 0,01 g |
| - Conservateur qs | |
| - Eau qsp | 100 g |

**EXEMPLE 5**

On prépare un fond de teint de composition suivante :

A)

| | | |
|---|---|---|
| - Eau | 44,80 | g |
| - p-hydroxybenzoate de méthyle | 0,10 | g |
| - Propylèneglycol | 2,00 | g |
| - Oxyde de fer jaune | 1,00 | g |
| - Oxyde de fer rouge | 0,40 | g |
| - Oxyde de fer noir | 0,20 | g |
| - Oxyde de titane | 9,40 | g |
| - Mélanine obtenue par oxydation du 5,6-dihydroxy-indole | 0,01 | g |
| - Silicate de magnésium et hydrate d'aluminium | 1,00 | g |
| - Lauroyl sarcosinate de sodium dans l'eau à 30% vendu sous la dénomination "ORAMIX L30" par la Société SEPPIC | 0,18 | g MA |
| - Carboxyméthyl cellulose de sodium | 0,20 | g |
| - Triéthanolamine | 1,00 | g |

B)

| | | | |
|---|---|---|---|
| B1 | - Acide stéarique | 2,20 | g |
| B1 | - Mélange de mono-di-stéarate de glycéryle, acide stéarique, glycérine (40/50/5/5) vendu sous la dénomination "GELEOL" par la Société GATTEFOSSE | 2,20 | g |
| B1 | - Triglycérides d'acides caprylique vendu sous la dénomination "MIGLYOL 812" par la Société HÜLS | 15,00 | g |
| B1 | - p-hydroxybenzoate de propyle | 0,30 | g |
| | - Cyclopenta diméthylsiloxane | 10,00 | g |
| | - DL α-tocophérol (vitamine E) vendu par la Société HOFFMANN LA ROCHE | 0,09 | g |

| | |
|---|---|
| **C)** | |
| - Eau | 1,00 g |
| - Imidazolidinyl urée | 0,30 g |
| - Glycérine | 3,00g |
| **D)** | |
| - Parfum | 0,20 g |
| **E)** | |
| - Amidon réticulé par anhydride octénylsuccinique vendu sous la dénomination "DRY FLO PC" par la Société NATIONAL STARCH | 5,00 g. |

**MODE OPERATOIRE :**

1) Dans un bécher on chauffe à 95°C. On introduit le propylèneglycol et on dissout le parahydroxybenzoate de méthyle. A 80°C, on disperse les pigments pendant 1 heure, puis la mélanine pendant 10 minutes. Sous agitation, on introduit le silicate de magnésium et d'aluminium hydraté. On laisse refroidir à 70°C et on rajoute l'ORAMIX L30 ainsi que la carboxyméthylcellulose de sodium et la triéthanolamine.
2) On fond les ingrédients de la phase **B1** à 80°C. On y ajoute le cyclopentadiméthyl siloxane et la vitamine E. On maintient le mélange **B** à 65°C.
3) On verse la phase **B** sur la phase **A** sous agitation à la turbine. On laisse refroidir sous agitation jusqu'à 40°C puis on ajoute la phase **C**, préparée à une température inférieure à 40°C, puis on ajoute **D** et **E** sous agitation et on laisse revenir la température à 25°C.

**EXEMPLE 6**

On prépare un mascara de composition suivante :

| | | | |
|---|---|---|---|
| A | - Stéarate de triéthanolamine | 12,0 | g |
| | - Cire d'abeilles | 4,0 | g |
| | - Cire de carnauba | 1,5 | g |
| | - Paraffine | 6,0 | g |
| | - DL-α-tocophérol (Vitamine E) vendu par la Société HOFFMANN LA ROCHE | 0,36 | g |
| | - Oxyde de fer noir | 5,0 | g |
| | - Mélanine obtenue par oxydation du 5,6-dihydroxyindole | 0,04 | g |
| B | - Hydroxyéthyl cellulose | 1,5 | g |
| | - Gomme arabique | 1,0 | g |
| | - Polyvinylpyrrolidone | 1,0 | g |
| | - Parahydroxybenzoate de méthyle | 0,2 | g |
| | - Parahydroxybenzoate de propyle | 0,7 | g |
| | - Eau qsp | 100 | g |

**MODE OPERATOIRE :**

On chauffe les éléments de la phase **A** à 85°C, on ajoute les oxydes de fer et la mélanine puis on mélange à la turbine.

On fait bouillir l'eau de la préparation et y dissout les conservateurs, puis à 85°C, on ajoute les éléments de la phase **B**.

On ajoute la phase aqueuse à 85°C à la phase **A** (80°C) auquel on a introduit la vitamine E en mélangeant à la turbine. On refroidit en mélangeant jusqu'à 27°C.

**EXEMPLE 7**

On prépare une crème antisolaire de composition suivante :

| | |
|---|---|
| - Mélanine obtenue par oxydation du 5,6-dihydroxyindole | 0,08 g |
| - Mélange de tocophérols naturels dans de l'huile de soja (50/50) vendu sous la dénomination "TOCOPHEROL Concentrat Naturel" par la Société ROSSOW | 0,04 g |

(suite)

| | |
|---|---|
| - p-méthoxycinnamate de 2-éthylhexyle vendu sous la dénomination "PARSOL MCX" par la Société GIVAUDAN | 5,0 g |
| - Alcool cétylique (90% $C_{16}$) | 15,0 g |
| - Monostéarate de polyéthylèneglycol à 50 moles d'oxyde d'éthylène vendu sous la dénomination "MYRJ 53" par la Société ICI | 3,0 g |
| - Mono et distéarate de glycérol (40/50) vendu sous la dénomination "GELEOL" par la Société GATTEFOSSE | 3,0 G |
| - Huile de vaseline | 24,0 g |
| - Eau qsp | 100 g |

**EXEMPLES 8**

On prépare une crème de soins pour le visage de composition suivante :

***Première phase*** :

- Lipide amphiphile non-ionique de formule générale :

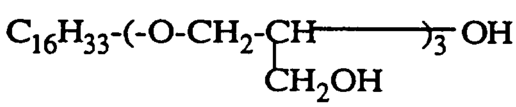 3,8 g

- Cholestérol 3,8 g
- Dicétylphosphate 0,4 g
- DL-α-tocophérol (Vitamine E) vendu par la Société HOFFMANN LA ROCHE 0,05 g
- Glycérol 3,0 g
- Mélanine obtenue par oxydation du 5,6-dihydroxy-indole 0,01 g
- Conservateurs qs
- Eau 52,44 g

| *Deuxième phase* : | |
|---|---|
| - Huile de macadamia | 15,0 g |
| - Parfum qs | |
| - Acide polyacrylique réticulé vendu sous la dénomination "CARBOPOL 940" par la Société GOODRICH | 0,4 g |
| - Triéthanolamine | 0,4 g |
| - Eau | 20,0 g |

On prépare la première phase de la manière suivante : on mélange les lipides à 95°C à l'état fondu. On y ajoute la

Vitamine E. On porte la phase aqueuse (glycérol + eau) à 80°C. On mélange une partie de la phase aqueuse à la phase lipidique sous faible agitation pour former une phase lamellaire. On ajoute le reste de la phase aqueuse pour constituer la phase de dispersion. On ajoute la mélanine et les conservateurs. On agite jusqu'à obtention d'une suspension vésiculaire concentrée. On homogénéise afin de réduire la taille des vésicules (diamètre moyen autour de 200 nm).

Puis on ajoute la deuxième phase, l'huile de macadamia étant d'abord dispersée par agitation mécanique à 40°C, puis le parfum, le "CARBOPOL 940", la triéthanolamine et l'eau étant incorporés.

**EXEMPLE 9**

on prépare une crème de soins pour le corps de composition suivante :

**1)** *Préparation d'une dispersion de nanoparticules*

Dans un bécher de 100 ml, on dissout 250 mg de condensat d'oxyde d'éthylène et d'oxyde de propylène (20/80%), de poids moléculaire moyen 1750 et 8350 respectivement, vendu sous la dénomination commerciale de "Pluronic F68" par la Société BASF, dans 50 ml d'eau déminéralisée, sous l'agitation donnée par un barreau magnétique tournant à 400 t/mn. Dans cette phase aqueuse thermostatée à la température de 40°C, on verse lentement 25 ml d'acétone, dans lesquels on a préalablement dissous, à la température de 55°C, 250 mg de lécithine de soja vendue sous la dénomination commerciale "EPIKURON 170" par la Société LUCAS MEYER, puis après être revenu à la température de 40°C, on dissout :

- 500 mg d'un mélange de tocophérol dans de l'huile de soja (50/50) vendu sous la dénomination "TOCOPHEROL Concentrat Naturel" par la Société ROSSOW.
- 125 mg de polycaprolactone commercialisé par la Société ALDRICH.

On maintient l'agitation pendant 2 heures à la température de 40°C, puis on revient à la température ambiante. On transfère alors la dispersion de nanoparticules obtenue dans un ballon rond de 250 ml que l'on place sur un évaporateur rotatif et on évapore l'acétone. on obtient ainsi une dispersion colloïdale, fluide de nanoparticules, dont le diamètre moyen est de 195 nm.

A l'examen microscopique en lumière blanche, on observe une population dense de nanoparticules, homogènes en tailles.

**2)** *Préparation d'une dispersion de vésicules*

Dans un bécher en verte de 100 ml, on pèse 0,05 g de dimyristyl phosphate et 0,95 g de tensio-actif non-ionique de formule (I) :

- Lipide non ionique de formule :

$$C_{12}H_{25}-\left[OC_2H_3(R)\right]-O-\left[C_3H_5-(OH)-O\right]_{\bar{n}}-H$$

formule dans laquelle :

* —$C_3H_5(OH)O$—
est constitué par un mélange de radicaux :

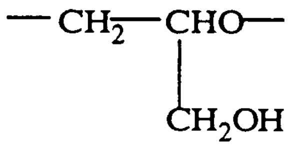

et

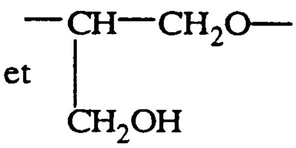

* $-O-C_2H_3(R)-$
  est constitué par un mélange de radicaux

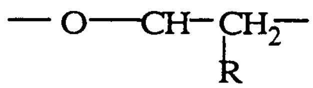

et

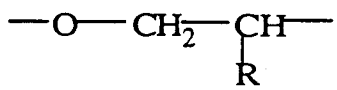

* $\overline{n}$ est une valeur statistique moyenne égale à 2,7 déterminée par RMN'H à 500 MHz,

* R est un mélange de radicaux $C_{14}H_{29}$ et $C_{16}H_{33}$

On homogénéise ces deux lipides par chauffage sur une plaque chauffante à la température de 100°C, puis on refroidit le mélange à 40°C. On ajoute alors 27,575 g d'eau dans laquelle on a préalablement dissous 3g de glycérine et 0,02 g d'acide citrique. On homogénéise le tout par action d'un ultradisperseur de type "VIRTIS" pendant 2 minutes à la vitesse de 40 000 t/mn, à la température de 40°C. On ajoute alors 3 g d'eau dans laquelle on a préalablement dissous 0,1 g de conservateur vendu sous la dénomination commerciale "KATHON CG" par la Société RHOM & HAAS et 0,2 g de conservateur vendu sous la dénomination commercial "GERMAL 115" par la Société RHOM & HAAS et 0,025 g de mélanine obtenue par oxydation du 5,6-dihydroxyindole.

On ajoute ensuite la phase grasse composée du mélange des produits suivants:

| | |
|---|---|
| - Huile de macadamia | 9,0 g |
| - Huile de silicone volaille | 7,0 g |
| - Paraméthoxy cinnamate d'éthyle hexyle vendu sous la dénomination commerciale "PARSOL MCX" par la Société GIVAUDAN | 0,5 g |
| - 2-hydroxy 4-méthoxybenzophénone vendu sous la dénomination commerciale "UVINUL M 40" par la Société BASF | 0,5 g |
| - Parahydroxybenzoate de propyle | 0,05 g |

On soumet le tout à l'action de l'ultradisperseur "VIRTIS" pendant 5 minutes à la température ambiante.

**3)** *Mélange des dispersions de vésicules et de nanoparticules*

On ajoute à la dispersion de vésicules obtenue 20 g de la dispersion aqueuse de nanoparticules préparée précédemment. On ajoute ensuite 35 g d'eau dans laquelle on a fait gonfler 0,65 g d'acide carboxyvinylique vendu sous la dénomination commerciale "CARBOPOL 940" par la Société GOODRICH. Après homogénéisation, on ajoute enfin 0,65 g de triéthanolamine diluée par 1,73 g d'eau. On obtient ainsi une crème blanche, épaisse, d'aspect brillant destinée au soin du corps. Après une application de cette crème, une fois par jour pendant quinze jours, on observe une amélioration de l'état de surface de la peau traitée.

## Revendications

1. Composition cosmétique caractérisée par le fait qu'elle comprend l'association d'au moins un tocophérol choisi parmi l'α-tocophérol, le β-tocophérol, le γ-tocophérol, le δ-tocophérol et leurs isomères, en une quantité comprisé entre 0,02 et 10% en poids, avec au moins un pigment mélanique dérivé de sources naturelles ou synthétiques, dans un support cosmétiquement acceptable.

2. Composition cosmétique selon la revendication 1, caractérisée en ce que le tocophérol est la vitamine E.

3. Composition cosmétique selon la revendication 1 ou 2, caractétisée par le fait que les pigments mélaniques sont présents dans la composition à une concentration comprise entre 0,001 et 2% en poids, et de préférence entre 0,05 et 0,5% en poids par rapport en poids total de la composition.

4. Composition selon la revendication 1, 2 ou 3, caractérisée en ce que le(s) tocophérols est (sont) présent(s) dans la composition à une concentration comprise entre 0,02 et 6% en poids par rapport au poids total de la composition.

5. Composition cosmétique selon l'une quelconque des revendications 1 à 4, caractérisée par le fait que les pigments mélaniques sont obtenus par oxydation d'au moins `un composé indolique, par polymérisation oxydante ou enzymatique de précurseurs mélaniques, par extraction de la mélanine de substances naturelles ou par culture de microorganismes produisant de la mélanine.

6. Composition cosmétique selon l'une des revendications 1 à 5, caractérisée par le fait que les pigments mélaniques sont obtenus par oxydation d'au moins un composé indolique de formule :

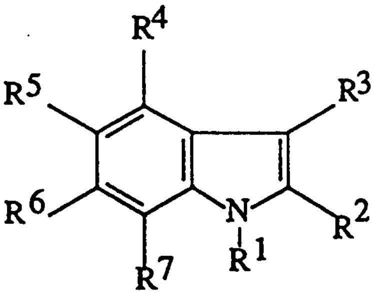

(I)

dans laquelle :

- $R^1$ et $R^3$ représentent, indépendamment l'un de l'autre, un atome d'hydrogène ou un groupe alkyle en $C_1$-$C_4$, $R^2$ représente un atome d'hydrogène, un groupe alkyle en $C_1$-$C_4$, un groupe carboxyle ou un groupe alcoxy ($C_1$-$C_4$)-carbonyle; $R^4$ et $R^7$ représentent, indépendamment l'un de l'autre, un atome d'hydrogène, un groupe hydroxy, un groupe alkyle en $C_1$-$C_4$, un groupe amino, un groupe alcoxy en $C_1$-$C_4$, un groupe acyl ($C_2$-$C_4$)-oxy, ou un groupe acyl ($C_2$-$C_4$)-amino; $R^5$ représente un atome d'hydrogène, un groupe hydroxy, un groupe alcoxy en $C_1$-$C_4$, un groupe alkyle en $C_1$-$C_4$, un atome d'halogène, un groupe amino, un groupe acyl ($C_2$-$C_{14}$)- oxy, un groupe acyl ($C_2$-$C_4$)-amino ou un groupe triméthylsilyloxy; $R^6$ représente un atome d'hydrogène, un groupe hydroxy, un groupe alcoxy en $C_1$-$C_4$, un groupe amino, un groupe acyl ($C_2$-$C_4$)-oxy, un groupe acyl ($C_2$-$C_4$)-amino, un groupe triméthylsilyloxy ou un groupe hydroxyalkyl ($C_2$-$C_4$)-amino; $R^5$ et $R^6$ pouvant également former, conjointement avec les atomes de carbone auxquels ils sont rattachés, un cycle méthylènedioxy éventuellement substitué par un groupement alkyle en $C_1$-$C_4$ ou alcoxy en $C_1$-$C_4$, ou bien un cycle carbonyldioxy; au moins l'un des radicaux $R^4$ à $R^7$ représente un groupement OZ ou $NHR^0$, l'un au plus des radicaux $R^4$ à $R^7$ représentant $NHR^0$ et deux au plus des radicaux $R^4$ à $R^7$ représentant OZ et, dans le cas où Z représente un atome d'hydrogène, les deux groupes OH sont dans les positions 5 et 6; et au moins l'un des radicaux $R^4$ à $R^7$ représente un atome d'hydrogène, et dans le cas où un seul de ces radicaux représente un atome d'hydrogène, un seul radical parmi les radicaux $R^4$ à $R^7$ représente alors $NHR^0$ ou OZ, les autres radicaux représentant des groupes alkyle en $C_1$-$C_4$; le radical $R^0$ du groupement $NHR^0$ désignant un atome d'hydrogène, un groupement acyle en $C_2$-$C_4$ ou hydroxyalkyle en $C_2$-$C_4$, et le radical Z du groupement OZ désignant un atome d'hydrogène, un groupe acyle en $C_2$-$C_{14}$, alkyle en $C_1$-$C_4$, ou triméthylsilyle;

et les sels de métaux alcalins, alcalino-terreux, d'ammonium ou d'amines de ce composé indolique.

7. Composition cosmétique selon la revendication 6, caractérisée par le fait que le composé indolique est choisi parmi le 4-hydroxyindole, le 5-hydroxyindole, le 6-hydroxyindole, le 7-hydroxyindole, le 4-hydroxy-5-méthoxyindole, le 4-hydroxy 5-éthoxyindole, le 2-carboxy 5-hydroxyindole, le 5-hydroxy 6-méthoxyindole, le 6-hydroxy 7-méthoxyindole, le 5-méthoxy 6-hydroxyindole, le 5,6-dihydroxyindole, le N-méthyl 5,6-dihydroxyindole, le 2-méthyl 5,6-dihydroxyindole, le 3-méthyl 5,6-dihydroxyindole, le 2,3-diméthyl 5,6-dihydroxyindole, le 2-carboxy 5,6-dihydroxyindole, le 4-hydroxy 5-méthyl indole, le 2-carboxy 6-hydroxyindole, le 6-hydroxy N-méthylindole, le 2-éthoxycarbonyl 5,6-dihydroxyindole, le 4-hydroxy 7-méthoxy 2,3-diméthylindole, le 4-hydroxy 5-éthoxy N-méthylindole, le 6-hydroxy 5-méthoxy 2-méthylindole, le 6-hydroxy 5-méthoxy 2,3-diméthylindole, le 6-hydroxy 2-éthoxycarbonylindole, le 7-hydroxy 3-méthylindole, le 5-hydroxy 6-méthoxy 2,3-diméthylindole, le 5-hydroxy 3-méthylindole, le 5-acétoxy 6-hydroxyindole, le 5-hydroxy 2-éthoxycarbonylindole, le 6-hydroxy 2-carboxy 5-méthylindole, le 6-hydroxy 2-éthoxycarbonyl 5-méthoxyindole, le 6-N-β-hydroxyéthylaminoindole, le 4-aminoindole, le 5-aminoindole, le 6-aminoindole, le 7-aminoindole, le N-méthyl 6-β-hydroxyéthylaminoindole, le 6-amino 2,3-diméthylindole, le 6-amino 2,3,4,5-tétraméthylindole, le 6-amino 2,3,4-triméthylindole, le 6-amino 2,3,5-triméthylindole, le 6-amino 2,3,6-triméthylindole, le 5,6,-diacétoxyindole, le 5-méthoxy 6-acétoxyindole, le 5,6-diméthoxyindole, le 5,6-méthylènedioxyindole, le 5,6-triméthylsilyloxyindole, l'ester phosphorique du 5,6-dihydroxyindole, le 5,6-dibenzyloxyindole, et les sels d'addition de ces composés.

8. Composition cosmétique selon la revendication 6 ou 7, caractérisée par le fait que le composé indolique est le 5,6-dihydroxyindole.

9. Composition cosmétique selon l'une -quelconque des revendications 1 à 8, caractérisée par le fait que le pigment mélanique est un pigment mélanique composite synthétique déposé à la surface ou incorporé dans une charge particulaire inerte minérale ou organique.

10. Composition cosmétique selon la revendication 9, caractérisée par le fait que la charge particulaire est constituée par des particules minérales inertes de granulométrie inférieure à 20 000 nm ou de polymères naturels ou synthétiques, organiques ou inorganiques à réseau réticulé, cristallin ou amorphe, de poids moléculaire compris entre 5000 et 5 000 000.

11. Composition cosmétique selon la revendication 9, caractérisé en ce que la charge particulaire est constituée par des particules à structure lamellaire, organiques ou minérales, de dimension inférieure à 50 000 nm.

12. Composition cosmétique selon l'une quelconque des revendications 1 à 11, caractérisée par le fait qu'elle se présente sous forme de lotion, lotion épaissie, gel, dispersion vésiculaire, dispersion de nanoparticules, crème, lait, poudre, pommade, bâtonnet solide, mousse ou spray.

13. Composition cosmétique selon la revendication 12, caractérisée par le fait qu'elle contient en outre des adjuvants cosmétiques choisis parmi les corps gras, les solvants organiques, les silicones, les épaississants, les adoucissants, les filtres solaires UV-A, UV-B ou à bande large, les agents anti-mousses, les agents hydratants, les parfums, les conservateurs, les tensio-actifs, les charges, les séquestrants, les polymères anioniques, cationiques, non-ioniques, amphotères ou leurs mélanges, les propulseurs, les agents alcalinisants ou acidifiants, les colorants et les pigments d'oxyde métallique.

14. Composition cosmétique selon l'une quelconque des revendications 1 à 13, constituant une composition protectrice de l'épiderme humain contre les radicaux libres, caractérisée par le fait qu'elle se présente sous forme de suspension ou de dispersion dans des solvants ou des corps gras, sous forme d'émulsion, de crème ou lait, sous forme de pommade, sous forme de gel, sous forme de bâtonnet solide ou de mousse aérosol.

15. Composition cosmétique selon l'une quelconque des revendications 1 à 13 utilisée pour la protection des cheveux contre les radicaux libres, caractérisée par le fait qu'elle se présente sous forme de shampooing, de lotion, de gel ou composition à rincer, à appliquer avant, ou après shampooing, avant ou après coloration ou décoloration, avant, pendant ou après permanente ou défrisage, sous forme de lotion ou de gel coiffants ou traitants, de lotion ou gel pour le brushing ou la mise en plis, de laque pour cheveux, de composition de permanente ou de défrisage, de coloration ou décoloration des cheveux.

16. Composition cosmétique selon l'une quelconque des revendications 1 à 13 constituant un produit de maquillage

des cils, de sourcils ou de la peau, caractérisée par le fait qu'elle se présente sous forme de crème de traitement de l'épiderme, fond de teint, bâton de rouge à lèvres, fard à paupières, fard à joues, ligneur encore appelé "eye-liner", ou mascara et se présente sous forme solide ou pâteuse, anhydre ou aqueuse.

**17.** Composition cosmétique selon l'une quelconque des revendications 1 à 13 constituant un produit de maquillage des cheveux, caractérisée en ce qu'elle se présente sous forme solide ou pâteuse, anhydre ou aqueuse, sous forme d'émulsion eau-dans-huile ou huile-dans-eau ou sous forme d'une suspension ou d'un gel.

**18.** Composition cosmétique selon l'une quelconque des revendications 1 à 13 constituant un produit à usage bucco-dentaire, caractérisée par le fait qu'elle comporte en outre au moins un agent humectant, un agent de polissage, un agent actif ou un édulcorant.

**19.** Procédé de protection cosmétique de l'epiderme humain, des muqueuses et des cheveux contre les radicaux libres, caractérisé par le fait qu'il consiste à appliquer sur ceux-ci une quantité efficace d'une composition cosmétique selon l'une quelconque des revendications 1 à 18.

**20.** Procédé de protection des compositions cosmétiques, vis-à-vis des radicaux libres, caractérisé en ce que l'on incorpore aux compositions cosmétiques une quantité efficace de l'association tocophérol/pigment mélanique selon l'une des revendications 1 à 18.

**21.** Procédé de maquillage de la peau, ses phanères ou des cheveux, caractérisé en ce qu'on leur applique une composition selon l'une des revendications 1 à 17.

## Claims

**1.** Cosmetic composition characterized in that it comprises the combination of at least one tocopherol chosen from α-tocopherol, β-tocopherol, γ-tocopherol, δ-tocopherol and their isomers, in a quantity of between 0.02 and 10 % by weight, with at least one melaninlike pigment derived from natural or synthetic sources, in a cosmetically acceptable carrier.

**2.** Cosmetic composition according to Claim 1, characterized in that the tocopherol is vitamin E.

**3.** Cosmetic composition according to Claim 1 or 2, characterized in that the melaninlike pigments are present in the composition in a concentration of between 0.001 and 2 % by weight, and preferably between 0.05 and 0.5 % by weight, relative to the total weight of the composition.

**4.** Composition according to Claim 1, 2 or 3, characterized in that the tocopherol(s) is (are) present in the composition in a concentration of between 0.02 and 6 % by weight relative to the total weight of the composition.

**5.** Cosmetic composition according to any one of Claims 1 to 4, characterized in that the melaninlike pigments are obtained by oxidation of at least one indolelike compound, by oxidizing or enzymatic polymerization of melaninlike precursors, by extraction of melanin from natural substances or by culture of microorganisms which produce melanin.

**6.** Cosmetic composition according to one of Claims 1 to 5, characterized in that the melaninlike pigments are obtained by oxidation of at least one indolelike compound of formula:

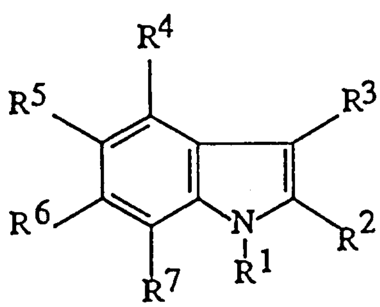

(I)

in which:

- $R^1$ and $R^3$ denote, independently of one another, a hydrogen atom or a $C_1$-$C_4$ alkyl group, $R^2$ denotes a hydrogen atom, a $C_1$-$C_4$ alkyl group, a carboxyl group or a $C_1$-$C_4$-alkoxycarbonyl group; $R^4$ and $R^7$ denote, independently of one another, a hydrogen atom, a hydroxyl group, a $C_1$-$C_4$ alkyl group, an amino group, a $C_1$-$C_4$ alkoxy group, a $C_2$-$C_4$ acyloxy group or a $C_2$-$C_4$ acylamino group; $R^5$ denotes a hydrogen atom, a hydroxyl group, a $C_1$-$C_4$ alkoxy group, a $C_1$-$C_4$ alkyl group, a halogen atom, an amino group, a $C_2$-$C_{14}$ acyloxy group, a $C_2$-$C_4$ acylamino group or a trimethylsilyloxy group; $R^6$ denotes a hydrogen atom, a hydroxyl group, a $C_1$-$C_4$ alkoxy group, an amino group, a $C_2$-$C_4$ acyloxy group, a $C_2$-$C_4$ acylamino group, a trimethylsilyloxy group or a $C_2$-$C_4$ hydroxyalkylamino group; it being also possible for $R^5$ and $R^6$ to form, together with the carbon atoms to which they are attached, a methylenedioxy ring optionally substituted by a $C_1$-$C_4$ alkyl or $C_1$-$C_4$ alkoxy group, or else a carbonyldioxy ring; at least one of the radicals $R^4$ to $R^7$ denotes a group OZ or $NHR^0$, not more than one of the radicals $R^4$ to $R^7$ denoting $NHR^0$ and not more than two of the radicals $R^4$ to $R^7$ denoting OZ and, in the case where Z denotes a hydrogen atom, the two OH groups are in positions 5 and 6; and at least one of the radicals $R^4$ to $R^7$ denotes a hydrogen atom, and in the case where only one of these radicals denotes a hydrogen atom, only one radical among the radicals $R^4$ to $R^7$ then denotes $NHR^0$ or OZ, the other radicals denoting a $C_1$-$C_4$ alkyl group; the radical $R^0$ of the group $NHR^0$ denoting a hydrogen atom, a $C_2$-$C_4$ acyl or $C_2$-$C_4$ hydroxyalkyl group, and the radical Z of the group OZ denoting a hydrogen atom, a $C_2$-$C_{14}$ acyl group, a $C_1$-$C_4$ alkyl group or a trimethylsilyl group;

and the alkali metal, alkaline-earth metal, ammonium or amine salts of this indolelike compound.

**7.** Cosmetic composition according to Claim 6, characterized in that the indolelike compound is chosen from 4-hydroxyindole, 5-hydroxyindole, 6-hydroxyindole, 7-hydroxyindole, 4-hydroxy-5-methoxyindole, 4-hydroxy-5-ethoxyindole, 2-carboxy-5-hydroxyindole, 5-hydroxy-6-methoxyindole, 6-hydroxy-7-methoxyindole, 5-methoxy-6-hydroxyindole, 5,6-dihydroxyindole, N-methyl-5,6-dihydroxyindole, 2-methyl-5,6-dihydroxyindole, 3-methyl-5,6-dihydroxyindole, 2,3-dimethyl-5,6-dihydroxyindole, 2-carboxy-5,6-dihydroxyindole, 4-hydroxy-5-methylindole, 2-carboxy-6-hydroxyindole, 6-hydroxy-N-methylindole, 2-ethoxycarbonyl-5,6-dihydroxyindole, 4-hydroxy-7-methoxy-2,3-dimethylindole, 4-hydroxy-5-ethoxy-N-methyl-indole, 6-hydroxy-5-methoxy-2-methylindole, 6-hydroxy-5-methoxy-2,3-dimethylindole, 6-hydroxy-2-ethoxycarbonyl-indole, 7-hydroxy-3-methylindole, 5-hydroxy-6-methoxy-2,3-dimethylindole, 5-hydroxy-3-methylindole, 5-acetoxy-6-hydroxyindole, 5-hydroxy-2-ethoxycarbonylindole, 6-hydroxy-2-carboxy-5-methylindole, 6-hydroxy-2-ethoxy-carbonyl-5-methoxyindole, 6-N-β-hydroxyethylaminoindole, 4-aminoindole, 5-aminoindole, 6-aminoindole, 7-amino-indole, N-methyl-6-β-hydroxyethylaminoindole, 6-amino-2,3-dimethylindole, 6-amino-2,3,4,5-tetramethylindole, 6-amino-2,3,4-trimethylindole, 6-amino-2,3,5-trimethyl-indole, 6-amino-2,3,6-trimethylindole, 5,6-diacetoxy-indole, 5-methoxy-6-acetoxyindole, 5,6-dimethoxyindole, 5,6-methylenedioxyindole, 5,6-trimethylsilyloxyindole, the phosphoric ester of 5,6-dihydroxyindole, 5,6-dibenzyloxyindole, and the addition salts of these compounds.

**8.** Cosmetic composition according to Claim 6 or 7, characterized in that the indolelike compound is 5,6-dihydroxyindole.

**9.** Cosmetic composition according to any one of Claims 1 to 8, characterized in that the melaninlike pigment is a synthetic composite melaninlike pigment deposited at the surface or incorporated into an inorganic or organic, inert particulate filler.

**10.** Cosmetic composition according to Claim 9, characterized in that the particulate filler consists of inert inorganic particles with particle size smaller than 20,000 nm or of organic or inorganic, natural or synthetic polymers with a crystalline or amorphous, crosslinked network, of molecular weight between 5000 and 5,000,000.

**11.** Cosmetic composition according to Claim 9, characterized in that the particulate filler consists of organic or inorganic particles of lamellar structure, smaller than 50,000 nm in size.

**12.** Cosmetic composition according to any one of Claims 1 to 11, characterized in that it is in the form of a lotion, thickened lotion, gel, vesicular dispersion, dispersion of nanoparticles, cream, milk, powder, ointment, solid stick, foam or spray.

**13.** Cosmetic composition according to Claim 12, characterized in that it additionally contains cosmetic adjuvants chosen from fatty substances, organic solvents, silicones, thickeners, emollients, UV-A, UV-B or broad band sunscreens, antifoam agents, hydrating agents, perfumes, stabilizers, surfactants, fillers, sequestrants, anionic, cationic, nonionic or amphoteric polymers or mixtures thereof, propellants, alkalifying or acidifying agents, dyes and metal oxide pigments.

**14.** Cosmetic composition according to any one of Claims 1 to 13, constituting a composition protecting the human epidermis against free radicals, characterized in that it is in the form of suspension or dispersion in solvents or fatty substances, the form of emulsion, of cream or milk, the form of ointment, the form of gel, the form of solid stick or of aerosol foam.

**15.** Cosmetic composition according to any one of Claims 1 to 13, employed for the protection of hair against free radicals, characterized in that it is in the form of shampoo, lotion, rinsing gel or composition, to be applied before or after shampooing, before or after dyeing or bleaching, before, during or after permanent waving or straightening, the form of styling or treating lotion or gel, of lotion or gel for blow drying or setting, of hair lacquer, or of permanent waving or hair straightening, dyeing or bleaching composition.

**16.** Cosmetic composition according to any one of Claims 1 to 13, constituting a make-up product for the eyelashes, the eyebrows or the skin, characterized in that it is in the form of treatment cream for the epidermis, make-up foundation, lipstick, eyeshadow, cheek blush, eyeliner or mascara and is in anhydrous or aqueous, solid or pasty form.

**17.** Cosmetic composition according to any one of Claims 1 to 13, constituting a make-up product for hair, characterized in that it is in anhydrous or aqueous, solid or pasty form, the form of water-in-oil or oil-in-water emulsion or the form of a suspension or of a gel.

**18.** Cosmetic composition according to any one of Claims 1 to 13, constituting a product for oral-dental care use, characterized in that it additionally comprises at least one moistening agent, a polishing agent, an active agent or a sweetener.

**19.** Process for protecting cosmetically the human epidermis, the mucosae and the hair against free radicals, characterized in that it consists in applying to the latter an effective quantity of a cosmetic composition according to any one of Claims 1 to 18.

**20.** Process for protecting cosmetic compositions against free radicals, characterized in that an effective quantity of the tocopherol/melaninlike pigment combination according to one of Claims 1 to 18 is incorporated in the cosmetic compositions.

**21.** Process for making up the skin, its apparent superficial growths or hair, characterized in that a composition according to one of Claims 1 to 17 is applied to them.

**Patentansprüche**

**1.** Kosmetische Zusammensetzung,
dadurch **gekennzeichnet,** daß
sie die Abmischung aus mindestens einem Tocopherol, ausgewählt aus α-Tocopherol, β-Tocopherol, γ-Tocopherol, δ-Tocopherol und aus deren Isomeren, in einer Menge von 0,02 bis 10 Gew.%, mit mindestens einem Melanin-Pigment, das aus natürlichen oder synthetischen Quellen stammt, in einem kosmetisch geeigneten Trägermittel umfaßt.

**2.** Kosmetische Zusammensetzung gemäß Anspruch 1,
dadurch **gekennzeichnet,** daß
das Tocopherol Vitamin E ist.

**3.** Kosmetische Zusammensetzung gemäß Anspruch 1 oder 2,
dadurch **gekennzeichnet,** daß
die Melanin-Pigmente in der Zusammensetzung mit einer Konzentration von 0,001 bis 2 und vorzugsweise von 0,05 bis 0,5 Gew.% vorhanden sind, bezogen auf das Gesamtgewicht der Zusammensetzung.

**4.** Zusammensetzung gemäß Anspruch 1, 2 oder 3,
dadurch **gekennzeichnet,** daß

die Tocopherole in der Zusammensetzung in einer Konzentration von 0,02 bis 6 Gew.% vorhanden sind, bezogen auf das Gesamtgewicht der Zusammensetzung.

5. Kosmetische Zusammensetzung gemäß jedem der Ansprüche 1 bis 4,
dadurch **gekennzeichnet,** daß
die Melanin-Pigmente durch Oxidation mindestens einer Indolverbindung, durch oxidierende oder enzymatische Polymerisation von Melanin-Vorstufenverbindungen, durch Extraktion des Melanins aus natürlichen Substanzen oder durch Züchtung von Melanin erzeugenden Mikroorganismen erhältlich sind.

6. Kosmetische Zusammensetzung gemäß einem der Ansprüche 1 bis 5,
dadurch **gekennzeichnet,** daß
die Melanin-Pigmente durch Oxidation mindestens einer Indolverbindung der Formel:

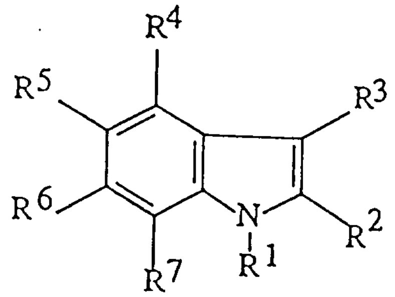

(I)

worin gilt:

$R^1$ und $R^3$ stellen, unabhängig voneinander, ein Wasserstoffatom oder eine $C_{1-4}$-Alkylgruppe dar; $R^2$ stellt ein Wasserstoffatom, eine $C_{1-4}$-Alkyl-, Carboxyl- oder eine $C_{1-4}$-Alkoxycarbonylgruppe dar; $R^4$ und $R^7$ stellen, unabhängig voneinander, ein Wasserstoffatom, eine Hydroxy-, $C_{1-4}$-Alkyl-, Amino-, $C_{1-4}$-Alkoxy-, $C_{2-4}$-Acyloxy- oder eine $C_{2-4}$-Acylaminogruppe dar; $R^5$ stellt ein Wasserstoffatom, eine Hydroxy-, $C_{1-4}$-Alkoxy-, $C_{1-4}$-Alkylgruppe, ein Halogenatom, eine Amino-, $C_{2-14}$-Acyloxy-, $C_{2-4}$-Acylamino- oder eine Trimethylsilyloxygruppe dar; $R^6$ stellt ein Wasserstoffatom, eine Hydroxy-, $C_{1-4}$-Alkoxy-, Amino-, $C_{2-4}$-Acyloxy-, $C_{2-4}$-Acylamino-, Trimethylsilyloxy- oder eine $C_{2-4}$-Hydroxyalkylaminogruppe dar; $R^5$ und $R^6$ können auch, zusammen mit den Kohlenstoffatomen, an die sie gebunden sind, einen gegebenenfalls mit einer $C_{1-4}$-Alkyl- oder $C_{1-4}$-Alkoxygruppe substituierten Methylendioxi- oder auch einen Carbonyldioxi-Ring bilden; mindestens einer der Reste $R^4$ bis $R^7$ stellt eine OZ- oder $NHR^0$-Gruppierung dar, wobei höchstens einer der Reste $R^4$ bis $R^7$ $NHR^0$ und höchstens zwei der Reste $R^4$ bis $R^7$ OZ darstellen und dabei, wenn Z ein Wasserstoffatom darstellt, die beiden OH-Gruppen dann in den Positionen 5 und 6 vorliegen; und mindestens einer der Reste $R^4$ bis $R^7$ stellt ein Wasserstoffatom dar, und, wenn nur einer dieser Reste ein Wasserstoffatom darstellt, stellt nur ein Rest unter den Resten $R^4$ bis $R^7$ dann $NHR^0$ oder OZ dar, wobei die anderen Reste dann eine $C_{1-4}$-Alkylgruppe darstellen, wobei der Rest $R^0$ der $NHR^0$-Gruppierung ein Wasserstoffatom, eine $C_{2-4}$-Acyl- oder eine $C_{2-4}$-Hydroxyalkylgruppe und der Rest Z der OZ-Gruppierung ein Wasserstoffatom, eine $C_{2-14}$-Acyl-, $C_{1-4}$-Alkyl- oder eine Trimethylsilylgruppe bedeuten,

oder durch Oxidation der Alkali-, Erdalkali-, Ammonium- oder Aminsalze dieser Indolverbindung erhältlich sind.

7. Kosmetische Zusammensetzung gemäß Anspruch 6,
dadurch **gekennzeichnet,** daß
die Indolverbindung aus 4-Hydroxyindol, 5-Hydroxyindol, 6-Hydroxyindol, 7-Hydroxyindol, 4-Hydroxy-5-methoxyindol, 4-Hydroxy-5-ethoxyindol, 2-Carboxy-5-hydroxyindol, 5-Hydroxy-6-methoxyindol, 6-Hydroxy-7-methoxyindol, 5-Methoxy-6-hydroxyindol, 5,6-Dihydroxyindol, N-Methyl-5,6-dihydroxyindol, 2-Methyl-5,6-dihydroxyindol, 3-Methyl-5,6-dihydroxyindol, 2,3-Dimethyl-5,6-dihydroxyindol, 2-Carboxy-5,6-dihydroxyindol, 4-Hydroxy-5-methylindol, 2-Carboxy-6-hydroxyindol, 6-Hydroxy-N-methylindol, 2-Ethoxycarbonyl-5,6-dihydroxyindol, 4-Hydroxy-7-methoxy-2,3-dimethylindol, 4-Hydroxy-5-ethoxy-N-methylindol, 6-Hydroxy-5-methoxy-2-methylindol, 6-Hydroxy-5-methoxy-2,3-dimethylindol, 6-Hydroxy-2-ethoxycarbonylindol, 7-Hydroxy-3-methylindol, 5-Hydroxy-6-methoxy-2,3-dimethylindol, 5-Hydroxy-3-methylindol, 5-Acetoxy-6-hydroxyindol, 5-Hydroxy-2-ethoxycarbonylindol, 6-Hydroxy-2-carboxy-5-methylindol, 6-Hydroxy-2-ethoxycarbonyl-5-methoxyindol, 6-N-β-Hydroxyethylaminoindol, 4-Aminoindol, 5-Aminoindol, 6-Aminoindol, 7-Aminoindol, N-Methyl-6-β-hydroxyethylaminoindol, 6-Amino-2,3-dimethylindol, 6-Amino-2,3,4,5-tetramethylindol, 6-Amino-2,3,4-trimethylindol, 6-Amino-2,3,5-trimethylindol, 6-Amino-2,3,6-trimethylindol, 5,6-Diacetoxyindol, 5-Methoxy-6-acetoxyindol, 5,6-Dimethoxyindol, 5,6-Methylendioxyindol, 5,6-Trimethylsilyloxyindol, Phosphorester von 5,6-Dihydroxyindol, 5,6-Dibenzyloxyindol sowie aus den Additionssalzen dieser Verbindungen ausgewählt.

**8.** Kosmetische Zusammensetzung gemäß Anspruch 6 oder 7,
dadurch **gekennzeichnet,** daß
die Indolverbindung 5,6-Dihydroxyindol ist.

**9.** Kosmetische Zusammensetzung gemäß jedem der Ansprüche 1 bis 8,
dadurch **gekennzeichnet,** daß
das Melanin-Pigment ein synthetisches Verbund-Melanin-Pigment ist, das auf der Oberfläche eines teilchenförmigen inerten mineralischen oder organischen Trägermittels abgeschieden oder darin eingebracht ist.

**10.** Kosmetische Zusammensetzung nach Anspruch 9,
dadurch **gekennzeichnet,** daß
das teilchenförmige Trägermittel aus mineralischen inerten Partikeln einer Korngröße von weniger als 20000 nm oder aus natürlichen oder synthetischen, organischen oder anorganischen Polymeren mit vernetztem, kristallinen oder amorphen Harzgerüst eines Molekulargewichts von 5000 bis 5000000 zusammengesetzt ist.

**11.** Kosmetische Zusammensetzung nach Anspruch 9,
dadurch **gekennzeichnet,** daß
das teilchenförmige Trägermittel aus organischen oder mineralischen Partikeln mit lamellarer Struktur mit einer Größe von weniger als 50000 nm zusammengesetzt ist.

**12.** Kosmetische Zusammensetzung gemaß jedem der Ansprüche 1 bis 11,
dadurch **gekennzeichnet,** daß
sie in Form einer Lotion, verdickten Lotion, eines Gels, einer bläschenartigen Dispersion, einer Dispersion aus Nanopartikeln, einer Creme, Milch, eines Pulvers, einer Pomade, eines Feststoffstäbchens, eines Schaums oder Spray vorliegt.

**13.** Kosmetische Zusammensetzung gemaß Anspruch 12,
dadurch **gekennzeichnet,** daß
sie ausserdem kosmetische Hilfsstoffe enthält, ausgewählt aus Fettkörpern, organischen Lösungsmitteln, Siliconen, Verdickungsmitteln, Weichmachern, Sonnenfilterstoffen für UV-A, UV-B oder für den langwelligen Bandenbereich, aus Antischaummitteln, Hydratisiermitteln, Parfüm-Produkten, Konservierungsstoffen, oberflächenaktiven Mitteln, Beaufschlagungsmitteln, Sequestriermitteln, anionischen, kationischen, nicht-ionischen und amphoteren Polymeren oder aus deren Mischungen, aus Treibmitteln, alkalisch oder sauer stellenden Mitteln, Farbstoffen und aus Pigmenten aus Metalloxiden.

**14.** Kosmetische Zusammensetzung gemaß jedem der Ansprüche 1 bis 13,
welche eine Zusammensetzung zum Schutz der menschlichen Epidermis vor freien Radikalen darstellt,
dadurch **gekennzeichnet,** daß
sie in Form einer Suspension oder Dispersion in Lösungsmitteln oder Fettkörpern, in Form einer Emulsion, Creme oder Milch, in Form einer Pomade, in Form eines Gels, in Form eines Feststoffstäbchens oder Aerosol-Schaums vorliegt.

**15.** Kosmetische Zusammensetzung gemäß jedem der Ansprüche 1 bis 13 zur Verwendung zum Schutz der Haare vor freien Radikalen,
dadurch **gekennzeichnet,** daß
sie in Form eines Shampoos in Form einer Lotion, eines Gels oder einer Zusammensetzung zur Spülung, zur Aufbringung vor oder nach dem Schamponieren, vor oder nach einer Färbung, vor, während oder nach einer Dauerwelle oder einem Entfrisiervorgang, in Form einer Lotion oder eines Gels zur Frisur oder sonstigen Behandlung, einer Lotion oder eines Gels zum Ausbürsten oder zur Wellengebung, eines Haarlacks, einer Zusammensetzung zur Dauerwelle oder zum Entfrisieren, zur Färbung oder Entfärbung der Haare vorliegt.

**16.** Kosmetische Zusammensetzung gemaß jedem der Ansprüche 1 bis 13,

welche ein Produkt zum Schminken der Wimpern, Augenbrauen oder der Haut darstellt, dadurch **gekennzeichnet,** daß sie in Form einer Creme zur Behandlung der Epidermis, einer Teint-Grundlage, eines Lippenstifts, von Lidschatten, Wangenschminken, eines Liniermittels, auch als "Eye-Liner" bezeichnet, oder einer Wimperntusche vorliegt und eine feste oder pasteuse wasserfreie oder wässrige Form aufweist.

**17.** Kosmetische Zusammensetzung gemäß jedem der Ansprüche 1 bis 13, welche eine Produkt zum Schminken der Haare darstellt, dadurch **gekennzeichnet,** daß sie in fester oder pasteuser, wasserfreier oder wässriger Form, in Form einer Wasser-in-Öl- oder Öl-in-Wasser-Emulsion oder in Form einer Suspension oder eines Gels vorliegt.

**18.** Kosmetische Zusammensetzung gemäß jedem der Ansprüche 1 bis 13, welche ein Produkt zur Verwendung im Mund- und/oder Dentalbereich darstellt, dadurch **gekennzeichnet,** daß sie ausserdem mindestens ein Feuchtigkeits-, Polier-, Wirkstoff- oder ein Süßungsmittel aufweist.

**19.** Verfahren zum kosmetischen Schutz der menschlichen Epidermis, der Schleimhäute und der Haare vor freien Radikalen, dadurch **gekennzeichnet,** daß man auf diese eine wirksame Menge einer kosmetischen Zusammensetzung gemäß jedem der Ansprüche 1 bis 18 aufbringt.

**20.** Verfahren zum Schutz kosmetischer Zusammensetzungen vor freien Radikalen, dadurch **gekennzeichnet,** daß man in die kosmetischen Zusammensetzungen eine wirksame Menge der Abmischung aus Tocopherol/Melanin-Pigment gemäß jedem der Ansprüche 1 bis 18 einbringt.

**21.** Verfahren zum Schminken der Haut, von Hautbereichen oder der Haare, dadurch **gekennzeichnet,** daß man eine Zusammensetzung gemäß einem der Ansprüche 1 bis 17 zur Anwendung bringt.